# EUROPÄISCHE PATENTANMELDUNG

(11) **EP 1 157 675 A1**
(43) Veröffentlichungstag der Anmeldung: **28.11.2001**
(21) Anmeldenummer: 01112107.6
(22) Anmeldetag: 17.05.2001
(51) Int. Cl.: A61F 2/44

(54) **Befüllbare künstliche Bandscheibe**

(30) Priorität: 19.05.2000 DE 10024922
(71) Anmelder: MINDA, ROLAND, DR.MED., 39124 MAGDEBURG (DE)
(72) Erfinder: Minda, Roland, Dr.med., 39124 Magdeburg (DE); Schmidt, Harald, 39218 Schönebeck (DE)
(74) Vertreter: Leinung, Günter

(57) **Zusammenfassung**

Die Erfindung betrifft eine künstliche Bandscheibe in Form einer Bandscheibenkernprothese (20) zum Ersatz des Bandscheibenkernes, welche nach einem operativen Eingriff in den Zwischenwirbelraum bzw. Bandscheibenraum (16) eingesetzt wird.

Bei der geschaffenen künstlichen Bandscheibe handelt es sich um eine Bandscheibenkernprothese (20), welche aus einem flexiblen Hüllkörper (1) besteht, der vorzugsweise aus einem Kunststoff hergestellt ist, einen Stutzen (3) aufweist, in dem ein Befüll- und Verschlußventil Aufnahme findet, über welches der Hüllkörper (1) im implantierten Zustand mittels eines Fluids oder mittels Luft befüllt werden kann.
Die Bandscheibenkernprothese (20) ist dabei so ausgeführt, dass am äußeren Umfang des Hüllkörpers (1), in horizontaler Ebene, eine Stützmanschette (2) vorgesehen ist, die unmittelbar mit dem Hüllkörper (1) verbunden und so ausgeführt ist, dass diese gleichfalls faltbar und entfaltbar ist, um mit dem Hüllkörper (1) bei der Implantation der Bandscheibenkernprothese (20) in den Bandscheibenraum (16) verbracht werden zu können.

## Beschreibung

Die Erfindung betrifft eine künstliche Bandscheibe in Form einer Bandscheibenkernprothese zum Ersatz des Bandscheibenkernes, welche nach einem operativen Eingriff in den Zwischenwirbelraum bzw. Bandscheibenraum eingesetzt wird.

Es sind bereits Bandscheibenkernprothesen bekannt, so eine Bandscheibenkernprothese nach der US 3875595, bei der die Bandscheibenkernprothese mit einem Fluid befüllbaren Hohlkörper ausgebildet ist, der in einem unbefüllten Zustand dorsal in den Zwischenwirbelraum einführbar sein soll. Zum Schutz der benachbarten Nerven und der Gefäßstrukturen erfolgt die Einführung der erschlafften, unbefüllten Bandscheibenkernprothese durch ein transparentes Rohr, das bis an den rückwärtigen Bereich der Wirbelkörper heranreicht und vor dem Bandscheibenfaserring endet. Vor Einführen des Hüllkörpers werden in die Wirbelkörper vom Zwischenwirbelraum aus hohle Zentrierstifte eingepreßt, in die Fortsätze des Hohlkörpers zu dessen Positionierung und Lagesicherung eingreifen sollen, wenn dieser mit Fluid befüllt wird. Die vorgesehenen Fortsätze zur Arretierung und Positionierung des Hüllkörpers sind wohl der Grund dafür, daß derartige bekannte Bandscheibenkemprothesen keinen Eingang in die medizinische Praxis gefunden haben, da hier zusätzliche Belastungen für den Patienten auftreten.

Aus der EP 0 304 305 A 1 ist eine Bandscheibenkernprothese bekannt, die zwei Fluid befüllbare Hüllkörper umfaßt, die im Bereich der lateralen Kanten beidseitig der beschädigten Bandscheibe durch zwei Zugänge einsetzbar sind. Ein derartiges zweiteiliges Implantat kann zwangsläufig nicht das gleiche mechanische Verhalten für die federnd schwenkbewegliche Abstützung der beiden Wirbelkörper gegeneinander haben wie der zentral angeordnete, natürliche Bandscheibenkern.

Ferner sei auf die EP 0 277 282 A 1 verwiesen, die eine komplette Bandscheibenprothese offenbart, die den gesamten Zwischenwirbelraum ausfüllt und sowohl den Bandscheibenkern wie auch den diesen umgebenden Bandscheibenfaserring ersetzt. Diese bekannte Gelenkendoprothese umfaßt feste Lagerschalen, die als Verankerungselemente dienen und aus mehreren Lagen eines metallischen Drahtnetzes bestehen. Zwischen den Lagerschalen liegt ein kissenartiger, elastischer Hohlkörper mit einem geschlossenen Hohlraum, der mit einem fließfähigen, inkompressiven Medium gefüllt ist. Eine derartige komplette Bandscheibenkemprothese, die den gesamten Raum zwischen zwei benachbarten Wirbelkörpern ausfüllt, ist nur ventral mittels eines entsprechend umfangreichen operativen Eingriffes implantierbar.

Unter Beachtung dieser Nachteile wurde mit der DE 39 22 203 C 1 ein chirurgisches Instrument zur Implantation einer Bandscheibenkernprothese vorgestellt, bei dem die Bandscheibenkernprothese mit einem ein Ventil aufweisenden und mit einem Fluid befüllbaren Hüllkörper besteht, der in einem unbefüllten oder nur teilweise mit Fluid befüllten Zustand in einen Zwischenwirbelraum einführbar und dort mit Fluid befüllbar sein soll, um den Zwischenwirbelraum auszufüllen.

Der Vorteil dieser Lösung, daß mit dem vorgestellten Instrument eine Bandscheibenkernprothese dorsal in einen Zwischenwirbelraum verbracht werden kann, wird durch den Nachteil dieser Bandscheibenkernprothese aufgebraucht, da die alleinige Lageposition und umfängliche Sicherung allein durch den Bandscheibenfaserring gesichert wird. Dies hat zur Folge, daß bei einer prolabierten Bandscheibe der Bandscheibenfaserring gleichfalls zerstört ist und somit eine Öffnung aufweist, durch die die implantierte Bandscheibenprothese teilweise austreten kann, somit ein künstlicher Bandscheibenvorfall eintreten kann.

Der Erfindung liegt daher die Aufgabe zugrunde, eine künstliche Bandscheibe in Form einer Bandscheibenkernprothese zu entwickeln, welche dorsal in einen Zwischenwirbelraum bzw. Bandscheibenraum einsetzbar und so gestaltet ist, daß diese in ihrer implantierten Lage verbleibt und somit die Nachteile der bekannten Lösungen ausgeschlossen werden.

Erfindungsgemäß wird die Aufgabe mit den Merkmalen der Ansprüche 1 and 7 gelöst.

Bevorzugte Ausführungen und vorteilhafte Lösungen sind den Unteransprüchen zu entnehmen.

So wurde eine Bandscheibenkernprothese geschaffen, welche aus einem flexiblen Hüllkörper besteht, der vorzugsweise aus einem Kunststoff hergestellt ist, einen Stutzen aufweist, in dem ein Befüll- und Verschlußventil Aufnahme findet, über welches der Hüllkörper im implantierten Zustand mittels eines Fluids oder mittels Luft befüllt werden kann.
Die vorgestellte Bandscheibenkernprothese ist ferner so ausgeführt, daß am äußeren Umfang des Hüllkörpers, in horizontaler Ebene, eine Stützmanschette vorgesehen ist, die unmittelbar mit dem Hüllkörper verbunden und so ausgeführt ist, daß diese gleichfalls faltbar und entfaltbar ist, um mit dem Hüllkörper bei der Implantation der Bandscheibenkernprothese in den Bandscheibenraum verbracht werden zu können.

Bei der Stützmanschette handelt es sich um ein Element, welches vorzugsweise aus einem metallischen Material, aus Titan, Viton oder Karbon, hergestellt ist, da diese Materialien besonders geeignet sind, mit dem menschlichen Gewebe eine Verbindung einzugehen und somit keine negativen Auswirkungen mit sich bringen.
Dabei kann diese Stützmanschette aus einem vollflächigen Material oder auch aus einem Drahtgeflecht hergestellt sein, wobei erfindungswesentlich ist, daß diese Stützmanschette zickzackförmig faltbar und somit weitestgehend ziehharmonikaähnlich gestaltet ist.
Dies ist insbesondere von Vorteil, da durch die in zwei Ebenen faltbare Stützmanschette ein räumliches Volumen, wenn diese zusammengefaltet ist, erhält, welches sich nicht störend bei der Implantation der gesamten Bandscheibenkernprothese auswirkt, sondern vielmehr hier der wesentliche Vorteil liegt, daß beim Befüllen des Hüllkörpers die Stützmanschette sich entfalten und eine stabile Lage einnehmen kann, um den Hüllkörper radial und umfänglich zu stützen.
Während die Stützmanschette, welche ihrer größenmäßigen Auslegung dem Innendurchmesser bzw. dem Innenmaß des Bandscheibenfaserringes angepaßt ist, dem Hüllkörper die umfängliche Stützung verleiht und gleichzeitig daran hindert, sich über dieses Maß hinaus zu entfalten bzw. auszudehnen, ist die Bandscheibenkernprothese im übrigen so ausgeführt, daß sich der Hüllkörper vertikal, somit nach oben und unten frei entfalten kann und bei Befüllung den Bandscheibenzwischenraum derart ausfüllt, daß die äußeren Wandungen des Hüllkörpers sich an die Wandungen der benachbarten Wirbelkörper anlehnen.

Der zur vorgestellten Bandscheibenkernprothese gehörende Stutzen zur Befüllung des Hüllkörpers ist dabei so gestaltet, daß im Inneren des Stutzens zwei beabstandete Membranen vorgesehen sind, als eine innere und eine äußere Membran ausgeführt und somit eine Befüllkammer bereitstellen, in denen ein Dichtelement, in bevorzugter Ausführung als Kugel gestaltet, angeordnet ist. Die eingesetzte Kugel dient dabei als Verschlußelement der Befüllöffnung der äußeren Membran, während die innere Membran so ausgebildet ist, daß sie an ihrem äußeren Umfang angeordnete und radial verlaufende Durchtrittsöffnungen besitzt. Das Verschließen der Befüllöffhung geschieht in der Form, daß durch die Befüllung des Hüllkörpers in diesem ein Druck aufgebaut wird, so daß die innere Membran gleichfalls druckbeaufschlagt wird und mit ihrer äußeren Wandung auf die Kugel wirkt und diese gegen die Befüllöffnung der äußeren Membran drückt, somit diese verschlossen wird. Durch das vorhandene Druckpotential im Hüllkörper ist ein sicherer Abschluß gewährleistet, wobei das Befüll- und Verschlußventil gleichfalls so ausgebildet ist, daß an diesem entsprechende Zuführrohre oder Leitungen anschließbar sind, über denen entsprechendes Medium in den Hohlkörper nachgefördert werden kann.

Es gehört auch zur Erfindung, daß der Hüllkörper der vorgestellten Bandscheibenkernprothese vollflächig und somit vollumfanglich von einem Stützkörper umschlossen ist, welcher einmal aus der bereits beschriebenen Stützmanschette besteht, dem beidseitig, in vertikaler Richtung, balgförmige Halbschalen zugeordnet sind. Die obere als auch die untere Halbschale bestehen vorzugsweise aus dem gleichen Material wie die Stützmanschette, so daß sich bei Entfaltung des Hüllkörpers, wenn dieser befüllt wird, sich gleichfalls die Halbschalen balgförmig ausbilden und somit den Hüllkörper vollumfänglich umschließen.

Dabei ist wesentlich, daß die Halbschalen ein besseres Dehnverhalten besitzen als die Stützmanschette, was dadurch realisiert wird, daß die Halbschalen werkstoffseitig mit einem anderen Elastizitätsmodul ausgeführt sind als die Stützmanschette.

Mit nachfolgendem Ausführungsbeispiel soll die Erfindung näher erläutert werden.

Die dazugehörige Zeichnung zeigt in
- Figur 1:: die Einordnung einer Bandscheibenkernprothese mit Stützmanschette in einen Bandscheibenraum
- Figur 2:: eine Bandscheibenkernprothese mit Stützmanschette
- Figur 3:: eine Draufsicht nach Figur 2
- Figur 4:: eine Einzelheit X nach Figur 3, den Stutzen mit Befüll- und Verschlußventil
- Figur 5:: eine Draufsicht nach Figur 4
- Figur 6:: die Einordnung einer Bandscheibenkernprothese mit Stützkörper in einem Bandscheibenraum
- Figur 7:: eine Bandscheibenkernprothese mit Stützkörper

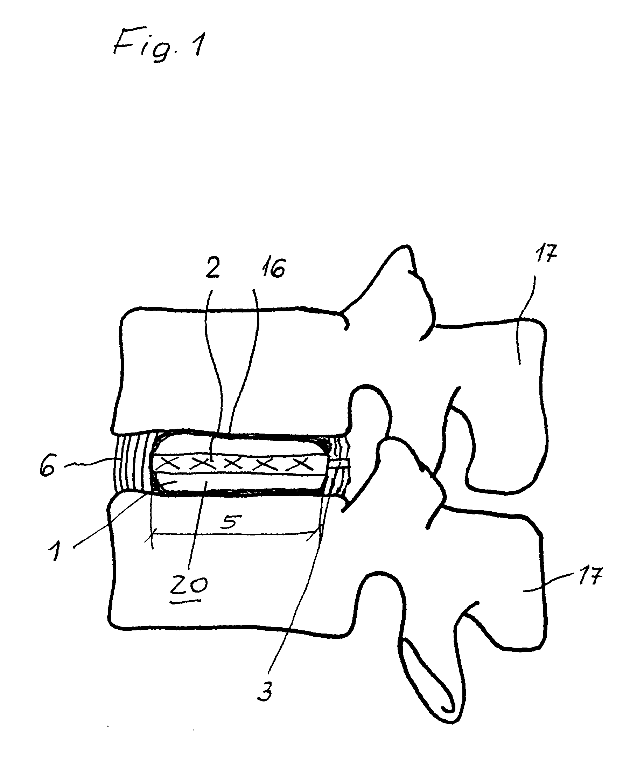

In der Figur 1 ist dargestellt, wie die Bandscheibenkernprothese 20 zwischen zwei Wirbelkörpern 17 in dem Bandscheibenraum 16 eingeordnet ist, wobei der Bandscheibenraum 16 umfänglich durch den Bandscheibenfaserring 6 weitestgehend begrenzt ist. Diese Darstellung verdeutlicht unmittelbar den Funktionszustand der Bandscheibenkernprothese 20, d. h. die Bandscheibenkernprothese 20 wurde operativ dorsal in den Bandscheibenraum 16 verbracht und der Hüllkörper 1 der Bandscheibenkernprothese 20 mit einem geeigneten Fluid bzw. mit einem Luftgemisch befüllt, was über den Stutzen 3 erfolgt, welcher mit einem Befüll- und Verschlußventil 4 versehen ist.
Aus der Darstellung nach Figur 1 ergibt sich ferner, daß die Bandscheibenkernprothese 20 mit einer Stützmanschette 2 ausgebildet ist, welche unmittelbar den Hüllkörper 1 in seinem Horizontalbereich 5 umfänglich umschließt.

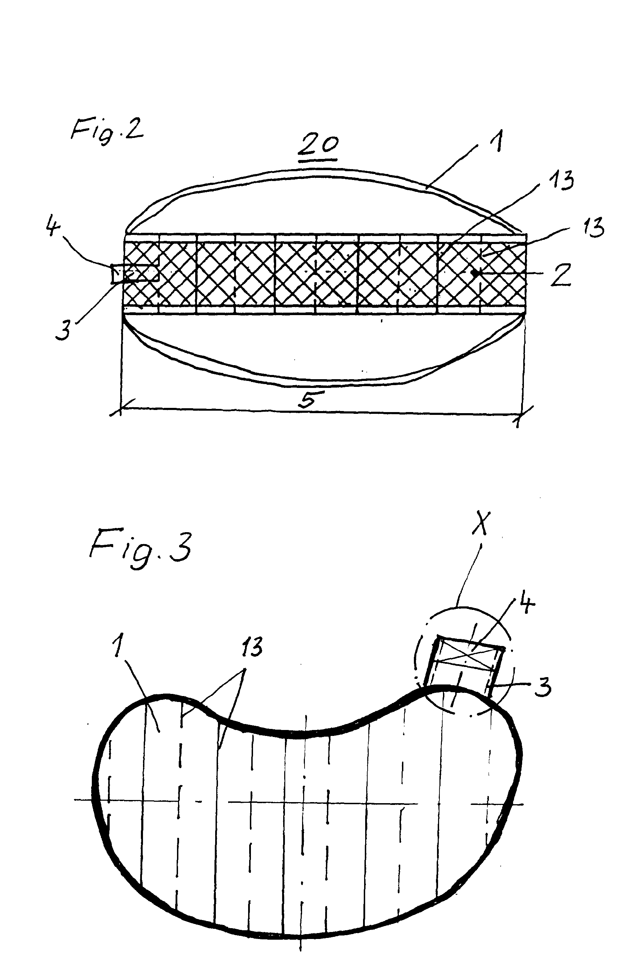

Die Darstellungen nach den Figuren 2 und 3 geben prinziphaft die Ausbildung und Gestaltung einer Bandscheibenkernprothese 20 wieder, wobei die Figur 2 im weitesten Sinne eine befüllte Bandscheibenkernprothese 20 darstellt, somit bei dieser Ausführung die einzelnen Elemente der Bandscheibenkernprothese 20 besser ersichtlich sind.

So ist gezeigt, daß der Hüllkörper 1, welcher aus einem flexiblen Material, vorzugsweise einem Kunststoff, besteht, von einer Stützmanschette 2 in seinem horizontalen Druckbereich 5, im größten Umfangsbereich des Hüllkörpers 1, umschlossen wird, wobei der Hüllkörper 1 mit der Stützmanschette 2 verbunden ist und als kompakte Einheit die Bandscheibenkernprothese 20 bestimmen. Die zeichnerische Darstellung der Stützmanschette 2 soll einmal verdeutlichen, daß die Stützmanschette 2 aus einem flächigen Material, aber auch aus einem Drahtgeflecht bzw. Drahtgewebe bestehen kann, wobei als Material vorzugsweise Titan, Viton oder Karbon zum Einsatz kommt.
Infolge der sehr beengten Freiheiten im Bereich zweier benachbarter Wirbelkörper 17 und des Bandscheibenraumes 16 sowie der Eintrittsöffnung durch den Bandscheibenfaserring 6 bedingen, daß die Bandscheibenkernprothese 20 in ihrer Gesamtheit beim Implantationsprozeß so ausgeführt bzw. gestaltet sein muß, daß sie ungehindert in den Bandscheibenraum 16 verbracht werden kann, ohne daß andere Körperteile beschädigt werden. Dies wird gesichert, indem die Stützmanschette 2 einmal durch die metallische "Dimensionierung" der Elemente der Stützmanschette ein "Zusammenlegen" gestattet, aber zusätzlich die Stützmanschette 2 so ausgeführt ist, daß sie zickzackförmig gefaltet werden kann, was mit den Bezugszahlen 13 gekennzeichnet ist.
Die dünnen Vollinien bedeuten dabei Erhebungen, während die gestrichelten Linien die Vertiefungen kennzeichnen.
Die Stützmanschette 2 ist dabei sowohl in vertikaler als auch in horizontaler Richtung zickzackförmig ausgebildet, so daß die Gewähr gegeben ist, daß die Stützmanschette 2 auf ein sehr kleines Volumen faltbar ist.
Das verwendete Material für den Hüllkörper 1 ist in seiner Beschaffenheit so ausgeführt, daß es gleichfalls zusammenlegbar und faltbar ist und andererseits gewährleistet, daß bei Befüllung des Hüllkörpers 1 sich der Hüllkörper 1 als auch die Stützmanschette 2 entfalten und ihre funktionsbedingte Form einnehmen.
Um die Bandscheibenkernprothese 20 befüllen, nachzufüllen bzw. entleeren zu können, ist ein Stutzen 3 vorgesehen, der mit einem Befüll- und Verschlußventil 4 ausgebildet ist, über den das entsprechende Medium in die Bandscheibenkernprothese 20 verbracht werden kann.

Der Stutzen 3 ist dabei allseitig dichtend mit dem Hüllkörper 1 verbunden und ferner so ausgebildet, daß er eingangsseitig mit entsprechenden Formgebungen ausgebildet ist, um Zuführleitungen zum Einbringen des Fluids oder des Luftgemisches anschließen zu können. Dies kann in Form von Steck-, Schraub- oder Klemmverschlüssen erfolgen.

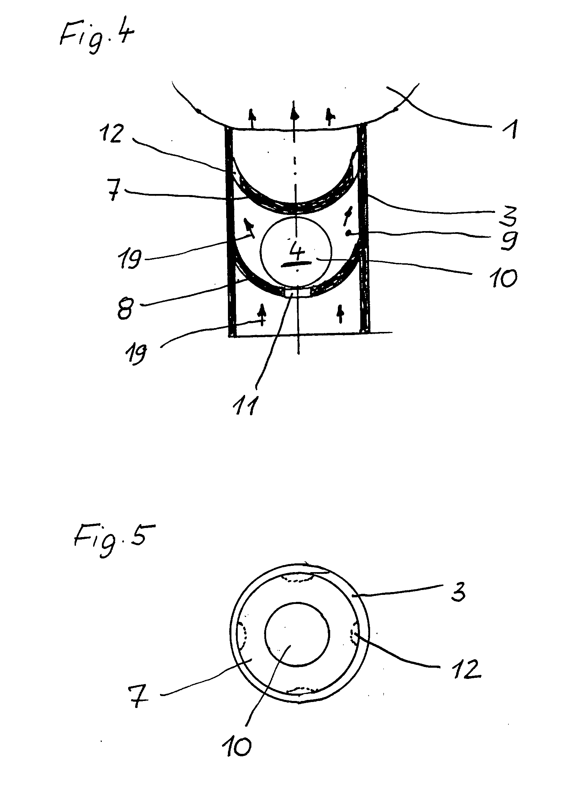

Die Ausbildung und Gestaltung des Befüll- und Verschlußventils 4 ist in den Figuren 4, 5 gezeigt, aus denen sich ergibt, daß innerhalb des Stutzens 3 zwei axial zueinander beabstandete Membranen 7, 8 vorgesehen sind, die zur Innenwandung des Stutzens 3 befestigt sind. Der Zwischenraum zwischen den beiden Membranen 7, 8 ist als Befüllkammer 9 gekennzeichnet, in der das Dichtelement 10 gleichfalls vorgesehen ist. Das Dichtelement 10 ist dabei vorzugsweise als eine Kugel ausgebildet, welche eine Verschluß-Dichtfunktion erfüllt.
Die äußere Membran 8 ist mit einer zentrischen Befilllöflhung 11 ausgeführt, während die innere Membran 7 Durchtrittsöffnungen 12 besitzt, die in Fließ- bzw. Strömungsrichtung 19 am äußeren Umfang der inneren Membran 7 vorgesehen und umfänglich verteilt sind.
Die Figur 5 verdeutlicht dabei die Anordnung und Ausbildung der Durchtrittsöffnungen 12 zwischen der Innenwandung des Stutzens 3 und dem äußeren Umfang der inneren Membran 7.

Die vorgesehenen Durchtrittsöffnungen 12 erfüllen dabei einmal die Funktion des Durchtrittes des einzufüllenden Mediums, andererseits gewährleisten sie einen entsprechenden Druckausgleich zwischen dem Inneren des Hüllkörpers 1 und der Befüllkammer 9, so daß die Verschluß-Dichtfünktion des Dichtelementes 10 gewährleistet ist.
Beim Befüllen des Hüllkörpers 1 gelangt das einzubringende Medium über die Befüllöffnung 11 in die Befüllkammer 9 und wird dann in Fließ- bzw. Strömungsrichtung 19 über die Durchtrittsöffnungen 12 geführt und gelangt danach in den Hüllkörper 1.
Liegt im Hüllkörper 1 der erforderliche Druck an, der für die Funktion der künstlichen Bandscheibe, der Bandscheibenkernprothese 20, erforderlich ist, wird die Zufuhr unterbrochen, und die innere Membran 7 drückt auf das Dichtelement 10 und verschiebt diese Kugel gegen die äußere Membran 8 derart, daß das Dichtelement 10 auf der Innenseite der äußeren Membran 8 im Bereich der Befüllöffnung 11 zum Anliegen kommt und diese verschließt.
Dabei ist es durchaus möglich, die Durchtrittsöffnungen 12 so zu gestalten, daß sie den Durchtritt des zuzuleitenden Mediums in Fließ- und Strömungsrichtung 19 gestatten, jedoch einen Austritt aus dem Hüllkörper 1 derart verhindern, daß die Durchtrittsöffnungen 12 mit Verschlußelementen ausgerüstet sind, die einen Austritt des Mediums aus dem Hüllkörper 1, entgegen der Fließ- bzw. Strömungsrichtung 19, verhindern. Dies kann beispielsweise über Klappsysteme realisiert werden.

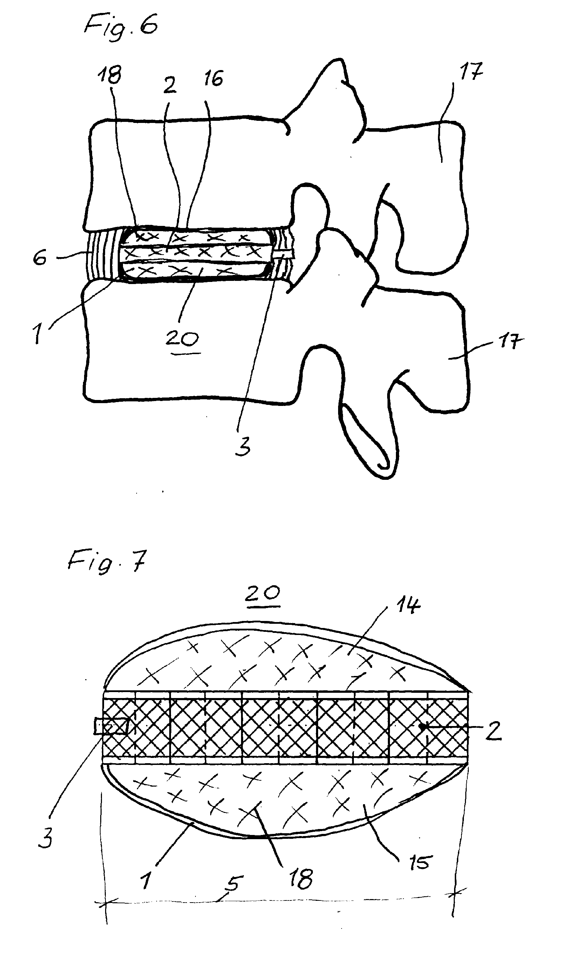

Eine weitere Ausführungsform einer Bandscheibenkernprothese 20 ist in der Figur 7 gezeigt, und die Darstellung nach der Figur 6 gibt wieder, wie eine derart ausgebildete Bandscheibenkernprothese 20 im Bandscheibenraum 16 eingeordnet ist.
Diese vorgestellte Bandscheibenkernprothese 20 ist so gestaltet, daß sie gleichfalls einen Hüllkörper 1 besitzt, welcher in der oben bereits beschriebenen Art und Weise in seinem horizontalen Druckbereich 5 mit einer Stützmanschette 2 umgeben ist und zusätzlich der Hüllkörper 1 im oberen und unteren Bereich mit einem Stützkörper 18 ausgebildet ist, welcher aus dem gleichen Material der Stützmanschette 2 besteht.
Der Stützkörper 18 besteht dabei aus zwei Halbschalen 14, 15, die als obere Halbschale 14 und als untere Halbschale 15 gekennzeichnet sind.
Dargestellt ist wieder die Bandscheibenkernprothese 20 in ihrem Funktionszustand, und es wird deutlich, daß die obere und untere Halbschale 14, 15 der Bandscheibenkernprothese 20 die balgförmige Ausbildung geben, die in ihrer funktionsbedingten Ausbildung dann der Form des Bandscheibenraumes 16 entspricht.
Bei dieser Ausbildung der Bandscheibenkernprothese 20 können die Halbschalen 14, 15 des Stützkörpers 18 so ausgebildet und angeordnet sein, daß sie den Hüllkörper 1 von außen umschließen und diesem die erforderliche Stützung verleihen, wobei es vorteilhaft ist, die Halbschalen 14, 15 des Stützkörpers 18 in den Hüllkörper 1 zu integrieren, vorzugsweise als ein Drahtgeflecht/Drahtgewebe auszubilden und einzuordnen, so daß bei Implantation dieser Bandscheibenkernprothese 20 kein "metallischer" Kontakt zu den Wirbelkörpern 17 gegeben ist, was sich vorteilhaft auf die Verträglichkeit auswirkt, wobei auch ein metallischer Kontakt, solange der Stützkörper 18 gleichfalls aus Titan, Viton oder Karbon hergestellt ist, keine Nachteile begründet.

Die Halbschalen 14, 15 des Stützkörpers 18 sind gleichfalls in ähnlicher Art der Stützmanschette 2 faltbar und entfaltbar, da auch die Halbschalen 14, 15 in horizontaler und vertikaler Richtung zickzackförmig faltbar sind, somit ziehharmonikaähnlich gefaltet und entfaltet werden können.
Funktionsbedingt ist diese Bandscheibenkernprothese 20 mit dem Stutzen 3 ausgebildet, welcher auch mit dem Befüll- und Verschlußventil 4 ausgerüstet ist.
In den gezeigten Darstellungen ist der Stutzen 3 so ausgebildet, daß er über die äußeren Abmaße der Bandscheibenkernprothesen 20 hinausragt, dies ist nur des besseren Verständnisses wegen gewählt, selbstverständlich kann der Stutzen 3 auch unmittelbar in den Hüllkörper 1 und somit in die jeweilige Bandscheibenkernprothese 20 eingedrückt werden.

Neben der Einbindung der Halbschalen 14, 15 vom Stützkörper 18 in den Hüllkörper 1 bzw. der äußeren Anordnung über dem Hüllkörper 1 ist auch denkbar und möglich, diese an den Innenwandungen des Hüllkörpers 1 vorzusehen.
Die balgförmige Ausbildung der mit einem Stützkörper 18 versehenen Bandscheibenkernprothese 20 wird dadurch unterstützt, daß die verwendeten Materialien für die Halbschalen 14, 15 mit einem Dehnverhalten ausgerüstet sind, das über dem Dehnverhalten der Materialien für die Stützmanschette 2 liegt, was werkstoffmäßig durch unterschiedliche Elastizitätsmodule erzielt bzw. bewirkt wird.

Die Ausbildung der vorgestellten Bandscheibenkernprothesen 20 gewährleistet einerseits, daß diese zu einem sehr kleinen Volumen zusammengelegt werden können, um problemlos in den jeweiligen Bandscheibenraum 16 eingesetzt werden zu können und andererseits gewährleistet, daß die Bandscheibenkernprothesen 20 sich nach der Implantation bei Befüllung mit dem entsprechenden Medium entfalten und eine Form annehmen, die der Form des Bandscheibenraumes 16 entspricht, dabei den benachbarten Wirbelkörpern 17 eine ausreichende Stützung zueinander geben, aber gleichzeitig so elastisch sind, daß die Beweglichkeit nicht eingeschränkt wird.
Ein weiterer Vorteil besteht darin, daß insbesondere durch die Ausbildung der Stützmanschette 2 gesichert ist, daß kein künstlicher Bandscheibenvorfall eintreten kann, indem nämlich der Hüllkörper 1 durch einen prolabierten Bandscheibenfaserring 6 austreten kann, wodurch die Funktion der künstlichen Bandscheibe dann verloren gehen würde.
Diesem wird durch die Ausbildung der Bandscheibenkernprothesen 20 entgegengewirkt, und die so geschaffenen Bandscheibenkernprothesen 20 sind sowohl Distanzhalter als auch Bewegungselement und in ihrer Funktions- und Wirkungsweise weitestgehend dem natürlichen Bandscheibenkern angepaßt.

## Patentansprüche

1. Künstliche Bandscheibe in Form einer Bandscheibenkernprothese, bestehend aus einem mit einem Fluid befüllbaren Hüllkörper, welche in einem unbefüllten Zustand in einen Zwischenraum zweier benachbarter Wirbel einführbar, mit einem Fluid befüllbar und mittels eines Verschlusses verschließbar ist, **dadurch gekennzeichnet, daß**
der flexible Hüllkörper (1) mit einer äußeren, den Hüllkörper (1) umfänglich in einem bestimmten Horizontalbereich (5) begrenzenden und umschließenden Stützmanschette (2) ausgebildet ist, welche falt- und entfaltbar ausgeführt und mit dem Hüllkörper (1) verbunden ist, wobei der Hüllkörper (1) einen Stutzen (3) besitzt, in dem ein Befüll- und Verschlußventil (4) angeordnet ist.

2. Künstliche Bandscheibe nach Anspruch 1, **dadurch gekennzeichnet, daß**
die Stützmanschette (2) den Hüllkörper (1) im befüllten Zustand mittig, in seinem größten Umfangsbereich umschließt und im entfalteten Zustand die äußere Form des Hüllkörpers (1) einnimmt, wobei die Stützmanschette (2) aus einem gewebeverträglichen Material, wie Titan, Viton oder Karbon, ausgebildet ist.

3. Künstliche Bandscheibe nach einem der Ansprüche 1 bis 2, **dadurch gekennzeichnet, daß**
die Stützmanschette (2) aus einem vollflächigen Material/Metallgeflecht bzw. Metallgewebe besteht und balgförmig, ziehharmonikaähnlich ausgeführt ist, wobei die Stützmanschette (2) in horizontaler und vertikaler Richtung im entspannten Zustand zickzackförmig ausgebildet ist.

4. Künstliche Bandscheibe nach einem der Ansprüche 1 bis 3, **dadurch gekennzeichnet, daß**
die äußeren Abmessungen des Hüllkörpers (1) mit Stutzen (3) im entfalteten Zustand mit der im Horizontalbereich (5) vorgesehenen Stützmanschette (2) dem Innenmaß des Bandscheibenfaserringes (6) entsprechen und die Stützmanschette (2) so dimensioniert ist, daß der Hüllkörper (1) im befüllten und entfalteten Zustand formschlüssig von dieser umschlossen wird.

5. Künstliche Bandscheibe nach einem der Ansprüche 1 bis 4, **dadurch gekennzeichnet, daß**
der Stutzen (3) der Bandscheibenkernprothese schlauchförmig gestaltet und mit zwei zueinander beabstandeten Membranen (7; 8) eine Befüllkammer (9) mit darin angeordnetem Dichtelement (10) bildend, ausgebildet ist, wobei die äußere Membran (8) eine zentrische Befüllöffnung (11) und die innere Membran (7) mehrere axial verlaufende Durchtrittsöffnungen (12) besitzen.

6. Künstliche Bandscheibe nach einem der Ansprüche 1 und 5, **dadurch gekennzeichnet, daß**
das Dichtelement (10) als eine Kugel ausgebildet ist, die im befüllten Zustand den Hüllkörper (1) nach außen verschließt.

7. Künstliche Bandscheibe in Form einer Bandscheibenkernprothese, bestehend aus einem mit einem Fluid befüllbaren Hüllkörper, welche in einem unbefüllten Zustand in einen Zwischenraum zweier benachbarter Wirbel einführbar, mit einem Fluid befüllbar und mittels eines Verschlusses verschließbar ist, **dadurch gekennzeichnet, daß**
der flexible Hüllkörper (1) voll umfänglich mit einem falt- und entfaltbaren Stützkörper (18) umschlossen ist, der balgförmig, ziehharmonikaähnlich ausgebildet ist und aus zwei annähernd schalenförmig ausgebildeten Halbschalen (14; 15), den Hüllkörper (1) im vertikalen Bereich, oben sowie unten, umgreifend und einer Stützmanschette (2), den Hüllkörper (1) im Horizontalbereich (5), umfänglich umgreifend, besteht und mit einem Stutzen (3) ausgeführt ist, wobei der Stützkörper (18) aus einem Material wie Titan, Viton oder Karbon besteht.

8. Künstliche Bandscheibe nach Anspruch 7, **dadurch gekennzeichnet, daß**
die Elastizitätsmodule der Halbschalen (14; 15) zu dem der Stützmanschette (2) unterschiedliche Werte besitzen, wobei die Halbschalen (14; 15) gegenüber der Stützmanschette (2) mit einem höheren Elastizitätsverhalten ausgeführt sind.
